# EUROPÄISCHE PATENTSCHRIFT

(11) **EP 3 119 840 B1**
(45) Veröffentlichungstag und Bekanntmachung des Hinweises auf die Patenterteilung: **31.10.2018**
(21) Anmeldenummer: 15711424.0
(22) Anmeldetag: 11.03.2015
(51) Int. Cl.: C09C 1/40, C09D 5/32, C08K 9/02, A61K 8/02, A61K 8/26, A61K 8/29, A61Q 1/02, C09C 1/00, C09D 11/037, C09D 17/00, C09D 5/36, C09D 5/28, C09D 7/40

(54) **EFFEKTPIGMENTE**
EFFECT PIGMENTS
PIGMENTS À EFFET

(30) Priorität: 20.03.2014 DE 102014003975
(43) Veröffentlichungstag der Anmeldung: 25.01.2017
(73) Patentinhaber: Merck Patent GmbH, 64293 Darmstadt (DE)
(72) Erfinder: DIETZ, Johann, 63128 Dietzenbach (DE); FOERDERER, Cornelia, 64646 Heppenheim (DE); HAFERKORN, Nicole, 64319 Pfungstadt (DE); PFAFF, Gerhard, 64839 Muenster (DE); MCFARLANE, Doreen, 64404 Bickenbach (DE)
(86) Internationale Anmeldenummer: PCT/EP2015/000536
(87) Internationale Veröffentlichungsnummer: WO 2015/139825

(56) Entgegenhaltungen:
- EP-A2- 0 681 009
- WO-A1-01/77235
- DE-A1- 19 618 563
- JP-A- 2005 075 941

## Beschreibung

Die Erfindung betrifft silberfarbene Effektpigmente mit einem starken Sparkle-Effekt basierend auf Al2O₃-Plättchen, die zwei hochbrechende Schichten aufweisen, sowie deren Verwendung insbesondere in Farben, Lacken, Druckfarben, Kunststoffen, als Dotiermittel für die Lasermarkierung von Kunststoffen und Papieren, als Additiv im Lebensmittel- und Pharmabereich und in kosmetischen Formulierungen.

Effektpigmente, die einen starken silbernen Glanzeffekt aufweisen und die nicht auf reinen Metallplättchen beruhen, sind von großem Interesse für verschiedene Anwendungen in Lacken, Kunststoffen, Druckfarben und Kosmetika. Zwar gibt es Pigmente in diesem Farbbereich bereits im Markt, wie z.B. Iriodin® 602 und 612 der Fa. Merck, doch weisen diese neben der silbernen Farbe keinen oder kaum Sparkle auf.

In der EP 0 246 523 B1 werden Perlglanzpigmente beschrieben, die eine eisen(II)-oxidhaltige Schicht aufweisen, wobei diese u. a. aus FeTiO₃ (Ilmenit) bestehen kann. Damit sind silberne, nichtmetallische Effektpigmente zugänglich. Die EP 0 681 009 A2 beschreibt die Verwendung von ilmenithaltigen Interferenzpigmenten zur Herstellung von fälschungssicheren Wertschriften und Verpackungen. In der WO 97/43348 A1 werden titanathaltige Perlglanzpigmente beschrieben, deren innere Schicht aus Titandioxidplättchen besteht. Die WO 2004/099319 A2 führt Interferenzpigmente mit hohem Deckvermögen aus, wobei auf ein plättchenförmiges, anorganisches Substrat mindestens eine FeTiO₃-haltige Schicht aufgebracht ist und der FeTiO₃-Anteil bezogen auf das Gesamtgewicht der Schicht 8 - 100 Gew.% beträgt und die Pigmente starke silberfarbene Effekte aufweisen können. In der WO 2012/130776 A1 werden hochglänzende silberfarbene Pigmente mit hoher Deckfähigkeit und metallischem Erscheinungsbild beschrieben, die auf einem nichtmetallischen plättchenförmigen synthetischen Substrat basieren und deren Gehalt an Eisenverbindungen im Pigment bei weniger als 5,0 Gew.-% bezogen auf das Gesamtgewicht des Pigmentes liegt. Aus der JP P2005-075941 A sind farbige metallische Pigmente bekannt, die eine Titanat-Schicht aufweisen.

Von Nachteil ist bei den im Stand der Technik beschriebenen nichtmetallischen Effektpigmente, dass sie je nach Zusammensetzung der Schichten zwar eine silberne Farbe besitzen können, sie aber keinen oder nur einen geringfügigen Sparkle zeigen.

Aufgabe der vorliegenden Erfindung ist es Effektpigmente bereit zu stellen, die sowohl einen sehr starken silberfarbenen Effekt mit hohem Glanz zeigen als auch einen hohen Sparkle aufweisen. Weiterhin sollten die Effektpigmente mittels eines einfachen Verfahrens herstellbar sein.

Überraschenderweise wurde nun gefunden, dass die Belegung von Al₂O₃-Plättchen mit einer genau definierten Partikelgröße mit mindestens zwei hochbrechenden Beschichtungen aus Titandioxid und FeTiO₃ (Ilmenit) zu silberfarbenen Effektpigmenten führt, die sich nicht nur durch ihre reine und silberne Körperfarbe verbunden mit einem starken Glanz auszeichnen, sondern auch durch ihren hohen Sparkle-Effekt.

Im Gegensatz zu den silbernen oder silbergrauen Effektpigmenten aus dem Stand der Technik zeigen die erfindungsgemäßen Pigmente
- eine reine silberne Körperfarbe
- einen sehr hellen und starken Glanz
- einen starken Sparkle-Effekt und
- ein hohes Deckvermögen.

Gegenstand der vorliegenden Erfindung sind daher Effektpigmente gemäß Anspruch 1, die sich dadurch auszeichnen, dass Al2O₃-Plättchen mit einer Dicke von < 500 nm mit einer Äquivalenzdurchmesserverteilung (D₉₀) nach der 90 % der Teilchen im Bereich von 5-45 µm liegen, auf der Oberfläche zwei hochbrechende Beschichtungen mit einer Brechzahl n von ≥ 1,9 aufweisen, wobei die Schichten aus Titandioxid und Ilmenit (FeTiO₃) bzw. TiO₂/Fe₂O₃ und FeTiO₃ bestehen.

Gegenstand der Erfindung ist weiterhin ein Verfahren zur Herstellung der erfindungsgemäßen Pigmente.

Gegenstand der vorliegenden Erfindung ist ebenfalls die Verwendung der erfindungsgemäßen Effektpigmente in Farben, Autolacken, Industrielacken, Autoreparaturlacken, Pulverlacken, Druckfarben, Kunststoffen, Knopfpasten, keramischen Materialien, Gläsern, zur Saatgutbeschichtung, als Additiv zum Laserschweißen von Kunststoffen, als Dotiermittel bei der Lasermarkierung oder beim Laserschweißen von Kunststoffen und Papieren, als Additiv zur Einfärbung im Lebensmittel- und Pharmabereich und in kosmetischen Formulierungen. Weiterhin sind die erfindungsgemäßen Pigmente auch zur Herstellung von Pigmentpräparationen sowie zur Herstellung von Trockenpräparaten, wie z. B. Granulaten, Chips, Pellets, Briketts, etc., geeignet. Die Trockenpräparate sind insbesondere für Druckfarben und für kosmetische Formulierungen geeignet.

Wesentlich für die erfindungsgemäßen silbernen Effektpigmente ist das Basissubstrat sowie die Schichtenfolge auf dem Substrat.

Zur Erzielung eines starken Sparkle-Effekts ist die genaue Einstellung der Partikelgröße des Ausgangssubstrats von Bedeutung. Unter Sparkle wird in dieser Anmeldung ein intensiver Glittereffekt verstanden.

Al₂O₃-Plättchen sowie deren Herstellung werden in der Literatur ausführlich beschrieben. So sind beispielsweise Al₂O₃-Plättchen bekannt aus den japanischen Patentanmeldungen JP 72572/1991 und JP 39362/1992.

Aus der US 5,702,519 sind mit TiO₂ dotierte Al₂O₃-Plättchen bekannt, die eine Dicke von < 1 µm aufweisen und einen Formfaktor (aspect ratio: Durchmesser/Dicke) von > 20 besitzen.

In der WO 2006/101306 A1 und WO 2008/026829 A1 werden mit Zink dotierte Al₂O₃-Plättchen offenbart.

Geeignete Al₂O₃-Plättchen als Substrat für die erfindungsgemäßen Effektpigmente können dotiert oder undotiert sein.

Sofern sie dotiert sind, handelt es sich bei der Dotierung vorzugsweise um TiO₂, ZrO₂, SiO₂, In₂O₃, SnO₂, oder ZnO bzw. deren Gemische. Vorzugsweise ist das Al₂O₃-Substrat undotiert oder mit TiO₂ dotiert.

Sofern das Al₂O₃-Substrat dotiert ist, beträgt der Anteil der Dotierung vorzugsweise < 5 %, insbesondere 0,05-3 % bezogen auf das Substrat.

Bei den Al₂O₃-Plättchen handelt es sich vorzugsweise um Korund.

Geeignete Al₂O₃-Plättchen sind vorzugsweise dotierte oder undotierte α-Al₂O₃-Plättchen, insbesondere mit TiO₂ dotierte α-Al₂O₃-Plättchen.

Für den Sparkle-Effekt ist insbesondere die Partikelgrößenverteilung der Al₂O₃-Plättchen von Bedeutung.

Geeignete Al₂O₃-Plättchen für die erfindungsgemäßen Effektpigmente besitzen eine Äquivalenzdurchmesser-Verteilung, nach der 90 % der Teilchen im Bereich von 5-45 µm, vorzugsweise 5-40 µm, liegen.

Die D₅₀-Werte liegen vorzugsweise im Bereich von 15-30 µm, ganz besonders bevorzugt im Bereich von 15-25 µm

Die D₁₀-Werte liegen vorzugsweise im Bereich von 5-15 µm, ganz besonders bevorzugt im Bereich von 6-10 µm.

In der gesamten Anmeldung werden die D₁₀-, D₅₀- und D₉₀-Werte bestimmt mit Malvern MS 2000.

Die Dicke der Al₂O₃-Plättchen beträgt < 500 nm, vorzugsweise 150-450 nm und insbesondere 150-400 nm.

Vorzugsweise beträgt der Formfaktor (aspect ratio: Durchmesser/Dicke-Verhältnis) der Al₂O₃-Plättchen 30-200, insbesondere 50-150.

Die Al₂O₃-Plättchen mit den oben definierten Partikelgrößen werden nachfolgend mit mindestens 2 hochbrechenden Schichten belegt.

Unter hochbrechend wird in dieser Anmeldung die Brechzahl n verstanden, die > 1,9 ist.

Auf das Substrat wird zunächst eine TiO₂-Schicht appliziert. Diese hochbrechende TiO₂-Beschichtung weist in der Regel Schichtdicken auf von 15 - 200 nm, vorzugsweise von 20 - 150 nm und insbesondere von 20 - 100 nm.

Das Titandioxid kann in der hochbrechenden Beschichtung in der Rutil- oder in der Anatasmodifikation vorliegen, vorzugsweise liegt es als Rutil vor. Die Verfahren zur Herstellung von Rutil sind im Stand der Technik beispielsweise beschrieben in der U.S. 5,433,779, U.S. 4,038,099, U.S. 6,626,989, DE 25 22 572 C2, EP 0 271 767 B1. Vorzugsweise wird vor der TiO₂-Auffällung auf das Al₂O₃-Plättchen eine dünne Zinnoxidschicht (< 10 nm) aufgebracht, die als Additiv dient um das TiO₂ in das Rutil zu überführen.

Bei der zweiten Schicht handelt es sich um eine farbige Schicht aus einem Gemisch aus TiO₂/Fe₂O₃ und FeTiO₃ (Ilmenit) oder einer reinen Ilmenit-Schicht. Vorzugsweise handelt es sich um eine Ilmenit-Schicht und keine Mischschicht aus TiO₂/Fe₂O₃ und FeTiO₃. Diese farbige Schicht befindet sich direkt auf der TiO₂-Schicht und weist in der Regel Schichtdicken von 5 - 100 nm, vorzugsweise von 8 - 80 nm und insbesondere von 8 - 50 nm auf.

Unter Schicht oder Beschichtung wird in dieser Anmeldung die vollständige Belegung des Substrats bzw. der ersten Schicht verstanden.

Die erfindungsgemäßen Effektpigmente lassen sich relativ leicht herstellen.

Die Belegung der Al₂O₃-Plättchen erfolgt vorzugsweise nasschemisch, wobei die zur Herstellung von Perlglanzpigmenten entwickelten nasschemischen Beschichtungsverfahren angewendet werden können. Derartige Verfahren sind z. B. beschrieben in DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017 oder auch in weiteren dem Fachmann bekannten Patentdokumenten und sonstigen Publikationen. Weiterhin kann die Beschichtung der Substrate auch in einem Wirbelbettreaktor durch Gasphasenbeschichtung erfolgen, wobei z. B. die in EP 0 045 851 A1 und EP 0 106 235 A1 zur Herstellung von Perlglanzpigmenten vorgeschlagenen Verfahren entsprechend angewendet werden können.

Die Belegung der Al₂O₃-Plättchen mit optional Zinnoxid und nachfolgend mit Titandioxid und Ilmenit bzw. einem TiO₂/Fe₂O₃-Gemisch erfolgt vorzugsweise nasschemisch, insbesondere nach dem Chlorid- oder Sulfatprozess.

Die Herstellung der erfindungsgemäßen Effektpigmente erfolgt in der Regel, indem die Al₂O₃-Plättchen in Wasser suspendiert und mit ein oder mehreren hdrolysierbaren Zinn-, Titan- und Eisensalzen bei einem für die Hydrolyse geeigneten pH-Wert versetzt werden, der so gewählt wird, dass die Metalloxide bzw. Metalloxidhydrate direkt auf den Plättchen ausgefällt werden, ohne dass es zu wesentlichen Nebenfällungen kommt. Der pH-Wert wird üblicherweise durch gleichzeitiges Zudosieren einer Base und/oder Säure konstant gehalten. Die beschichteten Substrate werden nach dem Filtrieren und Waschen zunächst 1 - 5 h bei Temperaturen von 50 - 150 °C, vorzugsweise bei 80 - 120 °C, getrocknet und nachfolgend bei 500 bis 1200 °C, vorzugsweise bei 500 - 1000 °C, insbesondere bei 500 - 800 °C, für 0,5 -5 h , vorzugsweise für 0,5 - 2 h unter reduzierten Bedingungen, vorzugsweise unter Formiergas (N₂/H₂), geglüht.

Die Zusammensetzung der FeTiO₃-haltigen Schicht wird durch das molare Verhältnis der zur Beschichtung einzusetzenden wasserlöslichen, anorganischen Titan- und Eisenverbindungen, jeweils berechnet als TiO₂ und Fe₂O₃, eingestellt. Das molare Verhältnis der eingesetzten Titan- und Eisenverbindungen, jeweils berechnet als TiO₂ und Fe₂O₃, beträgt vorzugsweise 1 : 0,1 bis 1 : 0,6, insbesondere 1 : 0,25 bis 1 : 0,5.

Die erhaltenen Effektpigmente mit den zwei hochbrechenden Schichten zeichnen sich durch reine silberne Farben, hohen Glanz, einen starken Sparkle-Effekt und hohes Deckvermögen aus.

Zur Erhöhung der Licht-, Wasser- und Wetterstabilität und/oder der Verbesserung der Benetzbarkeit und/oder Verträglichkeit empfiehlt es sich häufig, in Abhängigkeit vom Einsatzgebiet, das fertige silberweiße Effektpigment einer anorganischen oder organischen Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Durch diese Nachbeschichtung wird die chemische Stabilität weiter erhöht oder die Handhabung des Pigments, insbesondere die Einarbeitung in unterschiedliche Medien, erleichtert. Zur Verbesserung der Benetzbarkeit, Dispergierbarkeit und/oder Verträglichkeit mit den Anwendungsmedien können funktionelle Beschichtungen aus Al₂O₃ oder ZrO₂ oder deren Gemische bzw. Mischphasen auf die Pigmentoberfläche aufgebracht werden. Weiterhin sind organische, bzw. organisch/anorganisch kombinierte Nachbeschichtungen möglich, z.B. mit Silanen, wie beispielsweise beschrieben in der DE 10348174, EP 0090259, EP 0 342 533, EP 0 632 109, EP 0 888 410, EP 0 634 459, EP 1 203795, WO 94/01498, WO 96/32446, WO 99/57204, WO 2004/092284, U.S. 5,759,255, U.S. 5,571,851, WO 01/92425 oder in J.J. Ponjee, Philips Technical Review, Vol. 44, No. 3, 81 ff. und P.H. Harding J.C. Berg, J. Adhesion Sci. Technol. Vol. 11 No. 4, S. 471-493. Die Nachbeschichtung umfasst lediglich einen Gewichtsanteil von 0,1 bis 5 Gew. %, vorzugsweise 0,5 bis 3 Gew. %, bezogen auf das Effektpigment.

Da die erfindungsgemäßen silberfarbenen Effektpigmente neben einem hellen und starken Glanz einen starken Sparkle-Effekt zeigen, lassen sich mit ihnen besonders wirksame Effekte in den verschiedenen Anwendungsmedien erzielen.

Die erfindungsgemäßen Effektpigmente können zur Pigmentierung von Farben, Lacken, Druckfarben, Kunststoffen, kosmetischen Formulierungen, keramischen Materialien, Papier und Gläsern sowie in den verschiedenen Sicherheitsanwendungen in üblicher Weise eingesetzt werden. Weiterhin sind die erfindungsgemäßen Pigmente auch für die Lasermarkierung von Papier und Kunststoffen, für Anwendungen im Agrarbereich, sowie für die Herstellung von Pigmentpräparationen wie beispielsweise Pearlets, Pasten und Anteigungen und Trockenpräparaten, wie z.B. Pellets, Granulate, Chips, etc., die vorzugsweise in Druckfarben und Lacken verwendet werden, geeignet. Besonders geeignet sind die erfindungsgemäßen Pigmente für die Anwendung in Auto-, Autoreparatur- und Industrielacken. Sie lassen sich ebenso in einer Vielzahl der bekannten, in Farbsystemen angewendeten Bindemittel einsetzen und sind sowohl in wässrigen Systemen als auch in Systemen auf Lösungsmittelbasis anwendbar.

Es versteht sich von selbst, dass für die verschiedenen Anwendungszwecke die erfindungsgemäßen Effektpigmente auch vorteilhaft in Abmischung mit organischen Farbstoffen, organischen Pigmenten oder anderen Pigmenten, wie z. B. transparenten und deckenden Weiß-, Bunt- und Schwarzpigmenten sowie mit plättchenförmigen Eisenoxiden, organischen Pigmenten, holographischen Pigmenten, LCPs (Liquid Crystal Polymers), und herkömmlichen transparenten, bunten und schwarzen Glanzpigmenten auf der Basis von metalloxidbeschichteten Glimmer- und SiO₂-Plättchen, etc., verwendet werden können. Die Effektpigmente können in jedem Gewichtsverhältnis mit handelsüblichen Pigmenten und Füllstoffen gemischt werden. Vorzugsweise beträgt das Verhältnis 1 : 1 bis 9 : 1. Sofern die erfindungsgemäßen Effektpigmente mit Füllstoffen gemischt werden, kann das Mischungsverhältnis auch 99 : 1 bis 1 : 99 betragen.

In den verschiedenen Anwendungen kann das erfindungsgemäße Effektpigment auch mit weiteren Farbmitteln jeden Typs, z. B. organischen und/oder anorganischen Absorptionspigmenten und Farbstoffen, mehrschichtigen Interferenzpigmenten, wie z.B. Timiron®, Sicopearl® (BASF AG), ChromaFlair® (Flex Products Inc.), BiOCI-Pigmenten, Fischsilber, Metallpigmenten, z. B. von der Fa. Eckart, kombiniert werden. Dabei sind den Mischungsverhältnissen und Konzentrationen keine Grenzen gesetzt.

Die erfindungsgemäßen Effektpigmente sind mit einer Vielzahl von Farbsystemen kompatibel, vorzugsweise aus dem Bereich der Lacke, Farben und Druckfarben. Für die Herstellung der Druckfarben für z. B. den Tiefdruck, Flexodruck, Offsetdruck, Offsetüberdrucklackierung, ist eine Vielzahl von Bindern, insbesondere wasserlösliche Typen, geeignet, wie sie z. B. von den Firmen BASF, Marabu, Pröll, Sericol, Hartmann, Gebr. Schmidt, Sicpa, Aarberg, Siegberg, GSB-Wahl, Follmann, Ruco oder Coates Screen INKS GmbH vertrieben werden. Die Druckfarben können auf Wasserbasis oder Lösemittelbasis aufgebaut sein. Weiterhin sind die erfindungsgemäßen Effektpigmente auch für die Lasermarkierung von Papier und Kunststoffen, sowie für Anwendungen im Agrarbereich, z. B. für Gewächshausfolien, sowie z. B. für die Farbgebung von Zeltplanen, geeignet.

Die erfindungsgemäßen Effektpigmente können zur Pigmentierung von Lacken, Druckfarben, Kunststoffen, Agrarfolien, Saatgutbeschichtung, Knopfpasten, Arzneimittelüberzügen oder kosmetischen Formulierungen, wie Lippenstifte, Nagellacke, Presspuder, Shampoos, Seifen, lose Puder und Gele, verwendet werden. Die Konzentration des Pigments im zu pigmentierenden Anwendungssystem liegt in der Regel zwischen 0,1 und 70 Gew.%, vorzugsweise zwischen 0,1 und 50 Gew.% und insbesondere zwischen 0,5 und 10 Gew.%, bezogen auf den Gesamtfestkörpergehalt des Systems. Sie ist in der Regel abhängig vom konkreten Anwendungsfall.

In Kunststoffen enthaltend die erfindungsgemäßen Effektpigmente, vorzugsweise in Mengen von 0,01 bis 50 Gew.%, insbesondere 0,1 bis 7 Gew.%, lassen sich besonders ausgeprägte Silber- und Sparkle-Effekte erzielen.

Im Lackbereich, insbesondere im Automobillack, werden die erfindungsgemäßen Effektpigmente, auch für 3-Schichtaufbauten in Mengen von 0,1-20 Gew.%, vorzugsweise 1 bis 10 Gew.%, eingesetzt.

Im Lack haben die erfindungsgemäßen Effektpigmente den Vorteil, dass der angestrebte Glanz durch eine einschichtige Lackierung (Einschichtsystem bzw. Base coat im 2-Schichtaufbau) erzielt wird. Im Vergleich mit Lackierungen, die beispielsweise ein Mehrschichtpigment auf Basis von Glimmer bzw. ein herkömmliches, auf einem Substrat mit breiter Dickenverteilung basierendes Perlglanzpigment, statt der erfindungsgemäßen Effektpigmente enthalten, zeigen Lackierungen mit dem erfindungsgemäßen Pigment eine deutlichere Tiefenwirkung und einen stärker ausgeprägten Silber-, Glanz- und Sparkle-Effekt.

Die erfindungsgemäßen Effektpigmente können auch vorteilhaft in der dekorativen und pflegenden Kosmetik eingesetzt werden. Die Einsatzkonzentration reicht von 0,01 Gew.% im Shampoo bis zu 100 Gew. % bei losen Pudern. Bei einer Mischung der Pigmente mit Füllstoffen, vorzugsweise mit sphärischen Füllstoffen, wie z. B. SiO₂, kann die Konzentration bei 0,01-70 Gew.% in der Formulierung liegen. Die kosmetischen Produkte, wie z.B. Nagellacke, Presspuder, Shampoos, lose Puder und Gele, zeichnen sich durch besonders interessante Farbeffekte und einen hohen Glanz aus.

Die erfindungsgemäßen Pigmente können weiterhin mit handelsüblichen Füllstoffen gemischt werden. Als Füllstoffe sind z.B. zu nennen natürlicher und synthetischer Glimmer, Nylon Powder, reine oder gefüllte Melaminharze, Talkum, Gläser, Kaolin, Oxide oder Hydroxide von Aluminium, Magnesium, Calcium, Zink, BiOCI, Bariumsulfat, Calciumsulfat, Calciumcarbonat, Magnesiumcarbonat, Kohlenstoff, sowie physikalische oder chemische Kombinationen dieser Stoffe. Bezüglich der Partikelform des Füllstoffes gibt es keine Einschränkungen. Sie kann den Anforderungen gemäß z. B. plättchenförmig, sphärisch oder nadelförmig sein.

Selbstverständlich können die erfindungsgemäßen Effektpigmente in den Formulierungen auch mit jeder Art von kosmetischen Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z. B. Verdicker und rheologische Zusatzstoffe, wie z.B. Bentonite, Hektorite, Siliziumdioxide, Ca-Silicate, Gelatinen, hochmolekulare Kohlenhydrate und/oder oberflächenaktive Hilfsmittel etc.

Die die erfindungsgemäßen Effektpigmente enthaltenden Formulierungen können dem lipophilen, hydrophilen oder hydrophoben Typ angehören. Bei heterogenen Formulierungen mit diskreten wässrigen und nichtwässrigen Phasen können die erfindungsgemäßen Effektpigmente in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 5 und 8 liegen.

Den Konzentrationen der erfindungsgemäßen Effektpigmente in der Formulierung sind keine Grenzen gesetzt. Sie können - je nach Anwendungsfall - zwischen 0,001 (rinse-off-Produkte, z.B. Duschgele) und 100 % (z.B. Glanzeffekt-Artikel für besondere Anwendungen) liegen. Die erfindungsgemäßen Effektpigmente können weiterhin auch mit kosmetischen Wirkstoffen kombiniert werden. Geeignete Wirkstoffe sind z.B. Insect Repellents, UV A/BC-Schutzfilter (z.B. OMC, B3, MBC), Anti-Ageing-Wirkstoffe, Vitamine und deren Derivate (z.B. Vitamin A, C, E etc.), Selbstbräuner (z.B. DHA, Erytrolyse, u.a.) sowie weitere kosmetische Wirkstoffe wie z.B. Bisabolol, LPO, Ectoin, Emblica, Allantoin, Bioflavanoide und deren Derivate.

Bei der Pigmentierung von Bindemittelsystemen z. B. für Farben und Druckfarben für den Tiefdruck, Offsetdruck oder Siebdruck, oder als Vorprodukt für Druckfarben, hat sich der Einsatz der erfindungsgemäßen Effektpigmente in Form von hochpigmentierten Pasten, Granulaten, Pellets, etc., als besonders geeignet erwiesen. Die Effektpigmente werden in der Regel in die Druckfarbe in Mengen von 2-35 Gew.%, vorzugsweise 5-25 Gew.%, und insbesondere 8-20 Gew.% eingearbeitet. Offsetdruckfarben können die Pigmente bis zu 40 Gew.% und mehr enthalten. Die Vorprodukte für die Druckfarben, z.B. als Paste oder in Form von Granulaten, Pellets, Briketts, etc., enthalten neben dem Bindemittel und Additiven bis zu 98 Gew.% des erfindungsgemäßen Pigments. Die Druckfarben enthaltend die erfindungsgemäßen Pigmente zeigen reinere Farbtöne als mit herkömmlichen Silberpigmenten. Die Partikeldicken der erfindungsgemäßen Effektpigmente sind relativ gering und bedingen daher eine besonders gute Verdruckbarkeit.

Die erfindungsgemäßen Effektpigmente sind weiterhin geeignet zur Herstellung von fließfähigen Pigmentpräparationen und Trockenpräparaten, letztere z.B. in Form von Pellets, Granulaten, Chips, Briketts, Kugeln, Würstchen, vorzugsweise mit Teilchengrößen von 0,1 - 2 cm, insbesondere für Druckfarben, enthaltend ein oder mehrere erfindungsgemäße Effektpigmente, Bindemittel und optional ein oder mehrere Additive. Die Trockenpräparate können nach allen dem Fachmann bekannten Verfahren hergestellt werden, z.B. durch Granulieren, Sprühgranulieren, Sprühtrocknen, Pelletieren, Brikettieren, etc.

Gegenstand der Erfindung sind somit auch Formulierungen, insbesondere Autolacke, Autoreparaturlacke, Pulverlacke und Industrielacke, enthaltend die erfindungsgemäßen Effektpigmente.

Gegenstand der Erfindung sind insbesondere Formulierungen, die neben den erfindungsgemäßen Effektpigmenten mindestens einen Bestandteil ausgewählt aus Absorptionsmitteln, Adstringenzien, antimikrobiellen Stoffen, Antioxidantien, Antiperspirantien, Antischaummitteln, Antischuppenwirkstoffen, Antistatika, Bindemitteln, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Viskositätsreglern, Parfüm und Vitaminen enthalten.

Die nachfolgenden Beispiele sollen die Erfindung erläutern, ohne sie jedoch zu begrenzen.

Alle Prozentangaben, sofern nicht anders angegeben, sind Gewichtsprozent.

### Beispiele

### Beispiel 1

100 g Al₂O₃₋Plättchen der Teilchengröße 5-40 µm mit einer Dicke von 220-400 nm werden in 1 I entmineralisiertem Wasser unter Rühren auf 75 °C erhitzt. Anschließend wird mit einer 10 %igen Salzsäure der pH-Wert der Suspension auf 1,8 eingestellt. Es folgt das Zudosieren einer 2 %igen Zinntetrachloridlösung (5,6 g SnCl₄ (50 %) mit 17,2 g HCl 32 %ig und 110 ml entmineralisiertem Wasser gelöst), wobei der pH Wert durch gleichzeitiges Zutropfen einer 32 %igen Natronlauge konstant gehalten wird. Nach vollständiger Zugabe wird 15 min nachgerührt.
Anschließend folgt die Zugabe von 30 %iger Titantetrachloridlösung (68 g TiCl₄ (25 %) in 58,6 g entmineralisiertem Wasser gelöst), wobei der pH Wert durch gleichzeitiges Zutropfen einer 32 %igen Natronlauge konstant gehalten wird. Nach vollständiger Zugabe wird 15 min nachgerührt. Danach wird mit einer 32 %igen Natronlauge der pH-Wert der Suspension auf 2,8 gestellt. Im Anschluss werden 72,8 g einer FeCl₃-Lösung w=7,0 % zudosiert, wobei der pH-Wert durch gleichzeitiges Zutropfen einer 32 %igen Natronlauge konstant gehalten wird. Nach vollständiger Zugabe wird wieder 15 min nachgerührt. Es wird mit 32 %iger Natronlauge der pH-Wert von pH 5,0 eingestellt und nochmal 10 min nachgerührt.
Das Produkt wird abfiltriert, gewaschen, getrocknet, bei 650 °C 2 h unter reduzierenden Bedingungen (N₂/H₂) geglüht und durch ein Sieb gesiebt. Man erhält ein blaustichig silberfarbenes Effektpigment mit hohem Glanz und einem intensiven Sparkle-Effekt.

Die Partikelgrößenverteilung des Pigments nach Beispiel 1 bestimmt mit Malvern MS 2000 ergibt:
D₁₀ = 8,6 µm
D₅₀ = 18,2 µm
D₉₀ = 33,3 µm

### Beispiel 2

100 g Al₂O₃₋Plättchen der Teilchengröße 5-40 µm mit einer Dicke von 220-400nm werden in 1 I entmineralisiertem Wasser unter Rühren auf 75 °C erhitzt. Anschließend wird mit einer 10 %igen Salzsäure der pH-Wert der Suspension auf 1,8 eingestellt. Es folgt das Zudosieren einer 2 %igen Zinntetrachloridlösung (5,6 g SnCl₄ (50 %) mit 17,2 g HCl 32 %ig und 110ml entmineralisiertem Wasser gelöst), wobei der pH Wert durch gleichzeitiges Zutropfen einer 32 %igen Natronlauge konstant gehalten wird. Nach vollständiger Zugabe wird 15 min nachgerührt.
Anschließend erfolgt die Zugabe einer 30 %igen Titantetrachloridlösung (92 g TiCl4 (25 %) in 79,2 g entmineralisiertem Wasser gelöst), wobei der pH Wert durch gleichzeitiges Zutropfen einer 32 %igen Natronlauge konstant gehalten wird. Nach vollständiger Zugabe wird 15 min nachgerührt.
Danach wird mit einer 32 %igen Natronlauge der pH-Wert der Suspension auf 2,8 gestellt. Im Anschluss werden 72,8 g einer FeCl₃-Lösung w=7,0 % zudosiert, wobei der pH-Wert durch gleichzeitiges Zutròpfen einer 32 %igen Natronlauge konstant gehalten wird. Nach vollständiger Zugabe wird wieder 15min nachgerührt. Es wird mit 32 %iger Natronlauge der pH-Wert von pH 5,0 eingestellt und nochmal 10 min nachgerührt.
Das Produkt wird abfiltriert, gewaschen, getrocknet, bei 650 °C 2 h unter reduzierenden Bedingungen (N₂/H₂) geglüht und durch ein Sieb gesiebt. Man erhält ein neutral silberfarbenes Effektpigment mit hohem Glanz und einem intensiven Sparkle-Effekt.

Die Partikelgrößenverteilung des Pigments nach Beispiel 2 bestimmt mit Malvern MS 2000 ergibt:
D₁₀ = 8,8 µm
D₅₀ = 18,5 µm
D₉₀ = 33,5 µm

### Beispiel 3

100 g Al₂O₃₋Plättchen der Teilchengröße 5-40 µm mit einer Dicke von 220-400nm werden in 1 I entmineralisiertem Wasser unter Rühren auf 75 °C erhitzt. Anschließend wird mit einer 10 %igen Salzsäure der pH-Wert der Suspension auf 1,8 eingestellt. Es folgt das Zudosieren einer 2 %igen Zinntetrachloridlösung (5,6 g SnCl₄ (50 %) mit 17,2 g HCl 32 %ig und 110ml entmineralisiertem Wasser gelöst), wobei der pH Wert durch gleichzeitiges Zutropfen einer 32 %igen Natronlauge konstant gehalten wird. Nach vollständiger Zugabe wird 15 min nachgerührt. Nun wird eine 30 %ige Titantetrachloridlösung (120 g TiCl₄ (25 %) in 103,3 g entmineralisiertem Wasser gelöst) zugegeben, wobei der pH Wert durch gleichzeitiges Zutropfen einer 32 %igen Natronlauge konstant gehalten wird. Nach vollständiger Zugabe wird 15 min nachgerührt.
Anschließend wird mit einer 32 %igen Natronlauge der pH-Wert der Suspension auf 2,8 gestellt. Danach werden 72,8 g einer FeCl₃-Lösung w=7,0 % zudosiert, wobei der pH-Wert durch gleichzeitiges Zutropfen einer 32 %igen Natronlauge konstant gehalten wird. Nach vollständiger Zugabe wird wieder 15min nachgerührt. Es wird mit 32 %iger Natronlauge der pH-Wert von pH 5,0 eingestellt und nochmal 10 min nachgerührt.
Das Produkt wird abfiltriert, gewaschen, getrocknet, bei 650 °C 2 h unter reduzierenden Bedingungen (N₂/H₂) geglüht und durch ein Sieb gesiebt. Man erhält ein goldstichig silberfarbenes Effektpigment mit hohem Glanz und einem intensiven Sparkle-Effekt.

Die Partikelgrößenverteilung des Pigments nach Beispiel 3 bestimmt mit Malvern MS 2000 ergibt:
D₁₀ = 8,9 µm
D₅₀ = 18,6 µm
Dg₀ = 33,6 µm

### Beispiele 4-6: Nachbeschichtung

Zur Erhöhung der Licht-, Wasser- und/oder Wetterstabilität bzw. der verbesserten Einarbeitung der Pigmente in das jeweilige Anwendungsmedium werden die Effektpigmente der Beispiele 1-3 einer Nachbeschichtung unterzogen. Die Nachbeschichtung erfolgt wie in den nachfolgenden Patentanmeldungen und Patenten beschrieben:
DE 22 15 191, DE 31 51 354, DE 32 35 017, DE 33 34 598, DE10348174, EP 0090259, EP 0 342 533, EP 0632109, EP 0 888 410, EP 1203795, WO94/01498, WO 96/32446, WO 99/57204, WO 02/064682, WO 2004/092284 und US 5,759,255. Der Anteil der Nachbeschichtung bei den Pigmenten der Beispiele 1-3 beträgt jeweils 2 % bezogen auf das Effektpigment.

### Anwendungsbeispiele

### Beispiele A1: - Lacksystem

90 Gew. % Hydroglasur BG/S farblos (Wasserlack der Fa. Ernst Diegel GmbH)
10 Gew. % blaustichig silberfarbenes Effektpigment aus Beispiel 1 Lackieren durch Aufsprühen bei 80 °C
5 min vortrocknen bei 80 °C
20 min einbrennen bei 180 °C

Beispiel A2 erfolgt analog Beispiel A1, aber das unbeschichtete Pigment aus Beispiel 1 wird ersetzt durch das entsprechende nachbeschichtete Pigment von Beispiel 1.

### Beispiel B: - Kunststoff

1 kg Polystyrolgranulat werden in einem Taumelmischer mit 5 g Haftmittel gleichmäßig benetzt. Dazu werden dann 42 g Effektpigment aus Beispiel 2 zugegeben und 2 min lang gemischt. Dieses Granulat wird auf einer Spritzgießmaschine unter üblichen Bedingungen zu Stufenplättchen mit den Maßen 4 x 3 x 0,5 cm verarbeitet. Die Stufenplättchen zeichnen sich durch ihren ausgeprägten Sparkle-Effekt aus.

Die Produkte der Anwendungsbeispiele A1, A2 und B zeichnen sich durch ihren hohen Glanz, ihre Silberfarbe und einen intensiven Sparkle-Effekt aus.

## Patentansprüche

1. Effektpigmente, **dadurch gekennzeichnet, dass** Al₂O₃-Plättchen mit einer Äquivalenzdurchmesserverteilung, nach der 90 % der Teilchen im Bereich von 5-45 µm liegen, auf der Oberfläche zwei hochbrechende Beschichtungen mit einer Brechzahl n > 1,9 aufweisen, wobei die Schichten aus Titandioxid und Ilmenit (FeTiO₃) bzw. TiO₂/Fe₂O₃ und FeTiO₃ bestehen, und dass die TiO₂-Schicht eine Dicke von 15-200 nm besitzt.

2. Effektpigmente nach Anspruch 1, **dadurch gekennzeichnet, dass** der Formfaktor (aspect ratio: Durchmesser/Dicke-Verhältnis) der Al₂O₃-Plättchen 30-200 beträgt.

3. Effektpigmente nach Anspruch 1 oder 2, **dadurch gekennzeichnet, dass** es sich bei den Al₂O₃-Plättchen um Korund handelt.

4. Effektpigmente nach einem der Ansprüche 1 bis 3, **dadurch gekennzeichnet, dass** die Al₂O₃-Plättchen mit TiO₂, ZrO₂, SiO₂, ln₂O₃, SnO₂, oder ZnO bzw. deren Gemischen dotiert sind.

5. Effektpigmente nach einem oder mehreren der Ansprüche 1 bis 4, **dadurch gekennzeichnet, dass** der Anteil der Dotierung < 5 % bezogen auf das Substrat beträgt.

6. Effektpigmente nach einem oder mehreren der Ansprüche 1 bis 5, **dadurch gekennzeichnet, dass** die Al₂O₃-Plättchen auf der Oberfläche zunächst eine Schicht aus Titandioxid und nachfolgend eine FeTiO₃-Schicht aufweisen.

7. Effektpigmente nach einem oder mehreren der Ansprüche 1 bis 6, **dadurch gekennzeichnet, dass** die Schicht aus Titandioxid in der Rutilmodifikation vorliegt.

8. Effektpigmente nach einem oder mehreren der Ansprüche 1 bis 7, **dadurch gekennzeichnet, dass** die Ilmenit-Schicht eine Dicke von 5 - 100 nm besitzt.

9. Verfahren zur Herstellung der Effektpigmente nach einem der Ansprüche 1 bis 8, **dadurch gekennzeichnet, dass** die Beschichtung der Al₂O₃-Plättchen nasschemisch durch Fällung aus Metallsalzen in wässrigem Medium und nachfolgende Glühung in einer reduzierenden Gasatmosphäre erfolgt.

10. Verwendung der Effektpigmente nach einem der Ansprüche 1 bis 8 in Farben, Knopfpasten, Autolacken, Autoreparaturlacken, Pulverlacken, Druckfarben, Sicherheitsdruckfarben, Kunststoffen, keramischen Materialien, Gläsern, Papier, zur Saatgutbeschichtung, in Sicherheitsanwendungen, als Dotiermittel für die Lasermarkierung von Kunststoffen und Papieren, als Additiv zum Laserschweißen von Kunststoffen, in kosmetischen Formulierungen und zur Herstellung von Pigmentpräparationen und Trockenpräparaten.

11. Formulierungen enthaltend ein oder mehrere Effektpigmente nach einem oder mehreren der Ansprüche 1 bis 8.

12. Formulierungen nach Anspruch 11, dass sie zusätzlich mindestens einen Bestandteil ausgewählt aus der Gruppe der Absorptionsmitteln, Adstringenzien, antimikrobiellen Stoffen, Antioxidantien, Antiperspirantien, Antischaummitteln, Antischuppenwirkstoffen, Antistatika, Bindemitteln, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Viskositätsreglern, Parfüm und Vitaminen enthalten.

13. Pigmentpräparationen und Pasten enthaltend ein oder mehrere Bindemittel, optional ein oder mehrere Additive und ein oder mehrere Effektpigmente gemäß einem oder mehreren der Ansprüche 1 bis 8.

14. Trockenpräparationen in Form von Pellets, Granulaten, Chips, Briketts, Kugeln, Würstchen enthaltend ein oder mehrere Effektpigmente gemäß einem oder mehreren der Ansprüche 1 bis 8.

## Claims

1. Effect pigments, **characterised in that** Al₂O₃ flakes having an equivalence diameter distribution according to which 90% of the particles are in the range from 5-45 µm have on the surface two highly refractive coatings having a refractive index n > 1.9, where the layers consist of titanium dioxide and ilmenite (FeTiO₃) or TiO₂/Fe₂O₃ and FeTiO₃, and **in that** the TiO₂ layer has a thickness of 15-200 nm.

2. Effect pigments according to Claim 1, **characterised in that** the form factor (aspect ratio: diameter/thickness ratio) of the Al₂O₃ flakes is 30-200.

3. Effect pigments according to Claim 1 or 2, **characterised in that** the Al₂O₃ flakes are corundum.

4. Effect pigments according to one of Claims 1 to 3, **characterised in that** the Al₂O₃ flakes are doped with TiO₂, ZrO₂, SiO₂, In₂O₃, SnO₂, or ZnO or mixtures thereof.

5. Effect pigments according to one or more of Claims 1 to 4, **characterised in that** the proportion of the doping is < 5%, based on the substrate.

6. Effect pigments according to one or more of Claims 1 to 5, **characterised in that** the Al₂O₃ flakes have on the surface firstly a layer of titanium dioxide and subsequently an FeTiO₃ layer.

7. Effect pigments according to one or more of Claims 1 to 6, **characterised in that** the layer of titanium dioxide is in the rutile modification.

8. Effect pigments according to one or more of Claims 1 to 7, **characterised in that** the ilmenite layer has a thickness of 5 - 100 nm.

9. Process for the preparation of the effect pigments according to one of Claims 1 to 8, **characterised in that** the coating of the Al₂O₃ flakes is carried out by wet-chemical methods by precipitation from metal salts in aqueous medium and subsequent calcination in a reducing gas atmosphere.

10. Use of the effect pigments according to one of Claims 1 to 8 in paints, button pastes, automotive paints, automotive refinish paints, powder coatings, printing inks, security printing inks, plastics, ceramic materials, glasses, paper, for coating seed, in security applications, as dopant for the laser marking of plastics and papers, as additive for the laser welding of plastics, in cosmetic formulations and for the preparation of pigment preparations and dry preparations.

11. Formulations comprising one or more effect pigments according to one or more of Claims 1 to 8.

12. Formulations according to Claim 11, **characterised in that** that they additionally comprise at least one constituent selected from the group of the absorbents, astringents, antimicrobial substances, antioxidants, antiperspirants, antifoaming agents, antidandruff active compounds, antistatics, binders, biological additives, bleaches, chelating agents, deodorants, emollients, emulsifiers, emulsion stabilisers, dyes, humectants, film formers, fragrances, flavours, insect repellents, preservatives, anticorrosion agents, cosmetic oils, solvents, oxidants, vegetable constituents, buffer substances, reducing agents, surfactants, propellant gases, opacifiers, UV filters and UV absorbers, denaturing agents, viscosity regulators, perfume and vitamins.

13. Pigment preparations and pastes comprising one or more binders, optionally one or more additives and one or more effect pigments according to one or more of Claims 1 to 8.

14. Dry preparations in the form of pellets, granules, chips, briquettes, beads or sausages comprising one or more effect pigments according to one or more of Claims 1 to 8.

## Revendications

1. Pigments d'effet, **caractérisés en ce que** des flocons d'Al₂O₃ qui présentent une répartition de diamètres d'équivalence selon laquelle 90% des particules sont dans la plage de 5-45 µm comportent sur la surface deux revêtements hautement réfractifs qui présentent un indice de réfraction n > 1,9, où les couches sont constituées par du dioxyde de titane et de l'ilménite (FeTiO₃) ou du TiO₂/Fe₂O₃ et du FeTiO₃, et **en ce que** la couche en TiO₂ présente une épaisseur de 15-200 nm.

2. Pigments d'effet selon la revendication 1, **caractérisés en ce que** le facteur de forme (rapport d'aspect : rapport diamètre/épaisseur) des flocons d'Al₂O₃ est de 30-200.

3. Pigments d'effet selon la revendication 1 ou 2, **caractérisés en ce que** les flocons d'Al₂O₃ sont du corindon.

4. Pigments d'effet selon l'une des revendications 1 à 3, **caractérisés en ce que** les flocons d'Al₂O₃ sont dopés avec du TiO₂, du ZrO₂, du SiO₂, de l'In₂O₃, du SnO₂, ou du ZnO ou avec des mélanges de ceux-ci.

5. Pigments d'effet selon une ou plusieurs des revendications 1 à 4, **caractérisés en ce que** la proportion de dopant est < 5%, sur la base du substrat.

6. Pigments d'effet selon une ou plusieurs des revendications 1 à 5, **caractérisés en ce que** les flocons d'Al₂O₃ comportent sur la surface en premier lieu une couche en dioxyde de titane et ensuite, une couche en FeTiO₃.

7. Pigments d'effet selon une ou plusieurs des revendications 1 à 6, **caractérisés en ce que** la couche en dioxyde de titane est selon la modification rutile.

8. Pigments d'effet selon une ou plusieurs des revendications 1 à 7, **caractérisés en ce que** la couche en ilménite présente une épaisseur de 5 - 100 nm.

9. Procédé pour la préparation de pigments d'effet selon l'une des revendications 1 à 8, **caractérisé en ce que** le revêtement des flocons d'Al₂O₃ est mis en oeuvre au moyen de procédés de dépôt chimique par voie humide par précipitation à partir de sels métalliques dans un milieu aqueux et ensuite, par calcination dans une atmosphère de gaz réducteur.

10. Utilisation des pigments d'effet selon l'une des revendications 1 à 8 dans des peintures, des pâtes pour boutons, des peintures d'automobile, des peintures de finition ou de retouche d'automobile, des revêtements pulvérulents, des encres d'impression, des encres d'impression de sécurité, des matières plastiques, des matériaux de céramique, des verres, du papier, pour revêtir des semences, dans des applications de sécurité, en tant que dopant pour le marquage au laser de matières plastiques et de papiers, en tant qu'additif pour le soudage au laser de matières plastiques, dans des formulations cosmétiques et pour la préparation de préparations de pigments et de préparations sèches.

11. Formulations comprenant un ou plusieurs pigment(s) d'effet selon une ou plusieurs des revendications 1 à 8.

12. Formulations selon la revendication 11, **caractérisées en ce qu'**elles comprennent de façon additionnelle au moins un constituant qui est sélectionné parmi le groupe des absorbants, des astringents, des substances antimicrobiennes, des antioxydants, des antitranspirants, des agents anti-mousse, des composés actifs antipelliculaires, des antistatiques, des agents de liaison, des additifs biologiques, des agents de blanchiment ou décoloration, des agents chélatants, des déodorants, des émollients, des émulsifiants, des stabilisateurs d'émulsion, des colorants, des agents d'humidification, des agents filmogènes, des fragrances, des arômes, des insectifuges, des conservateurs, des agents anticorrosion, des huiles cosmétiques, des solvants, des oxydants, des constituants végétaux, des substances tampon, des agents réducteurs, des surfactants, des gaz propulseurs, des opacifiants, des filtres UV et des absorbeurs UV, des agents dénaturants, des régulateurs de viscosité, des parfums et des vitamines.

13. Préparations et pâtes de pigments comprenant un ou plusieurs agent(s) de liaison, en option un ou plusieurs additif(s) et un ou plusieurs pigment(s) d'effet selon une ou plusieurs des revendications 1 à 8.

14. Préparations sèches sous la forme de pastilles, de granules, de fragments, de briquettes, de perles ou de saucisses comprenant un ou plusieurs pigment(s) d'effet selon une ou plusieurs des revendications 1 à 8.
